# EUROPEAN PATENT SPECIFICATION

(11) **EP 4 417 146 B1**
(45) Date of publication and mention of the grant of the patent: **16.09.2026**
(21) Application number: 23220758.9
(22) Date of filing: 29.12.2023
(51) Int. Cl.: A61B 17/32, A61B 17/3207, A61B 17/22, A61B 17/3209, A61B 18/14, A61B 17/295

(54) **ANTERIOR MITRAL LEAFLET LACERATION DEVICE**
ANTERIORE MITRALSEGEL-WENDEVORRICHTUNG
DISPOSITIF DE LACÉRATION DES FEUILLETS MITRALES ANTÉRIEURS

(30) Priority: 17.02.2023 US 202363485751 P
(43) Date of publication of application: 21.08.2024
(73) Proprietor: Tendyne Holdings, Inc., St. Paul MN 55117 (US)
(72) Inventor: ABUNASSAR, Chad J., Alameda, CA 94501 (US); CANIC, Marko, 3303 Jegenstorf (CH)
(74) Representative: Gill Jennings & Every LLP

(56) References cited:
- WO-A1-2023/017417
- US-A1- 2006 074 484
- US-A1- 2022 022 940
- US-A1- 2022 233 210
- US-A1- 2022 346 827
- US-A1- 2023 010 485

## Description

### Background of the Disclosure

Valvular heart disease, and specifically aortic and mitral valve disease, is a significant health issue in the United States. Valve replacement is one option for treating heart valve diseases. Prosthetic heart valves, including surgical heart valves and collapsible/expandable heart valves intended for transcatheter aortic valve replacement ("TAVR") or transcatheter mitral valve replacement ("TMVR"), are well known in the patent literature. Surgical or mechanical heart valves may be sutured into a native annulus of a patient during an open-heart surgical procedure, for example. Collapsible/expandable heart valves may be delivered into a patient via a tube-like delivery apparatus such as a catheter, a trocar, a laparoscopic instrument, or the like to avoid a more invasive procedure such as full open-chest, open-heart surgery. As used herein, reference to a "collapsible/expandable" heart valve includes heart valves that are formed with a small cross-section that enables them to be delivered into a patient through a tube-like delivery apparatus in a minimally invasive procedure, and then expanded to an operable state once in place, as well as heart valves that, after construction, are first collapsed to a small cross-section for delivery into a patient and then expanded to an operable size once in place in the valve annulus.

Collapsible/expandable prosthetic heart valves typically take the form of a one-way valve structure (often referred to herein as a valve assembly) mounted to/within an expandable stent. In general, these collapsible/expandable heart valves include a self-expanding or balloon-expandable stent, often made of nitinol or another shape-memory metal or metal alloy (for self-expanding stents) or steel or cobalt chromium (for balloon-expandable stents). Existing collapsible/expandable TAVR devices have been known to use different configurations of stent layouts - including straight vertical struts connected by "V"s as illustrated in U.S. Pat. No. 8,454,685, or diamond-shaped cell layouts as illustrated in U.S. Pat. No. 9,326,856. The one-way valve assembly mounted to/within the stent includes one or more leaflets, and may also include a cuff or skirt. The cuff may be disposed on the stent's interior or luminal surface, its exterior or abluminal surface, and/or on both surfaces. A cuff helps to ensure that blood does not flow around the valve leaflets if the valve or valve assembly is not optimally seated in a valve annulus. A cuff, or a portion of a cuff, disposed on the exterior of the stent can help retard leakage around the outside of the valve (the latter known as paravalvular or "PV" leakage).

Balloon expandable valves are typically delivered to the native annulus while collapsed (or "crimped") onto a deflated balloon of a balloon catheter, with the collapsed valve being either covered or uncovered by an overlying sheath. Once the crimped prosthetic heart valve is positioned within the annulus of the native heart valve that is being replaced, the balloon is inflated to force the balloon expandable valve to transition from the collapsed or crimped condition into an expanded or deployed condition, with the prosthetic heart valve tending to remain in the shape into which it is expanded by the balloon. Typically, when the position of the collapsed prosthetic heart valve is determined to be in the desired position relative to the native annulus (*e.g*. via visualization under fluoroscopy), a fluid (typically a liquid although gas could be used as well) such as saline is pushed via a syringe (manually, automatically, or semi-automatically) through the balloon catheter to cause the balloon to begin to fill and expand, and thus cause the overlying prosthetic heart valve to expand into the native annulus.

There are several complications when implanting a prosthetic heart valve. For example, in the case of mitral valve repair, long, untethered, floppy anterior mitral leaflets (AMLs) represent an increased risk for systolic anterior motion (SAM) and consequent left ventricular outflow track obstruction in mitral valve replacement therapies. In some cases, around 10% of patients sent for assessment of certain mitral valve implantations are declined due to having an increased risk of developing systolic anterior motion and associated left ventricular outflow tract (LVOT) obstruction. On other occasions, a patient may be initially cleared for mitral valve replacement, but a risk develops between the time the patient was initially screened and the implantation timeframe as patient anatomy changes (e.g., chords rupture or elongate), and at the day of the procedure the physician may see an ad-hoc need for AML laceration to prevent SAM/LVOT obstruction.

Currently, the only procedure available to mitigate or prevent SAM is LAMPOON, a procedure in which AML is lacerated using traditional wires and catheters. A conventional LAMPOON procedure needs to be planned for in advance to valve implantation, and essentially be conducted prior a mitral valve replacement (e.g., a TENDYNE^{™} TMVI). However, this procedure is quite long, technically demanding for the surgeon, and is not always successful. In addition, since a LAMPOON procedure must be performed prior to implantation, any unpredictability of the anatomy's response to valve implantation poses additional risk of an unfavorable outcome. Some other LAMPOON procedures may be performed in valve-in-MAC or ring, or valve-in-valve procedures after the implantation as a bailout procedure. These also utilize traditional wires and/or catheters.

The disclosed device may be adapted further with a forward cutting orientation to alternatively be used to perform the BASILICA technique with a retrograde approach. In this other application, the cutting tool would lacerate aortic valve leaflets that obstruct coronary arteries after TAVI implantation.

US 2022/022940 A1 discloses a transcatheter heart valve leaflet cutter.

Among other advantages, it would be beneficial to provide new tools to achieve consistent and simpler leaflet laceration similar to LAMPOON or BASILICA procedures. Brief Summary of the Disclosure

The present invention is defined by the appended claims. Embodiments not falling within the scope of the claims are examples.

In some embodiments, a leaflet laceration device, includes an elongated shaft, a moveable arm coupled to the elongated shaft and pivotable relative thereto between an open condition and a closed condition, and a cutting element coupled to at least one of the elongated shaft and the moveable arm, the cutting element being configured and arranged to contact a first surface of an anterior mitral leaflet.

In some examples, a method of treatment, includes providing a leaflet laceration device including an elongated shaft, a moveable arm coupled to the elongated shaft and pivotable relative thereto between an open condition and a closed condition, and a cutting element coupled to at least one of the elongated shaft and the moveable arm, advancing the leaflet laceration device to an anterior mitral leaflet, and cutting the anterior mitral leaflet with the cutting element.

### Brief Description of the Drawings

FIGS. 1A-B are schematic cross-sectional illustrations of a heart showing normal left ventricular outflow tract (LVOT) flow during cardiac systole and LVOT obstruction.
FIGS. 2A-B are schematic illustrations showing one example of cutting an anterior mitral leaflet.
FIGS. 3A-C are schematic illustrations showing a retrograde approach via a femoral artery and aorta, an antegrade approach via a femoral vein, and a transapical approach.
FIG. 4 is a schematic illustration of a medical instrument for cutting a leaflet.
FIGS. 5A-C are schematic illustrations of one example of a laceration device according to a first embodiment.
FIGS. 6A-C are schematic illustrations of another example of a laceration device according to a second embodiment.
FIGS. 7A-C are schematic illustrations of another example of a laceration device according to a third embodiment.
FIGS. 8A-E are schematic illustrations of another example of a laceration device according to a fourth embodiment.

### Detailed Description

As used herein, the term "inflow end" when used in connection with a prosthetic heart valve refers to the end of the prosthetic valve into which blood first enters when the prosthetic valve is implanted in an intended position and orientation, while the term "outflow end" refers to the end of the prosthetic valve where blood exits when the prosthetic valve is implanted in the intended position and orientation. Thus, for a prosthetic aortic valve, the inflow end is the end nearer the left ventricle while the outflow end is the end nearer the aorta. For a prosthetic mitral valve, the inflow end is adjacent the atrium while the outflow end is the adjacent the ventricle. The intended position and orientation are used for the convenience of describing the valve disclosed herein, however, it should be noted that the use of the valve is not limited to the intended position and orientation, but may be deployed in any type of lumen or passageway. For example, although the prosthetic heart valve is described herein as a prosthetic aortic valve, the same or similar structures and features can be employed in other heart valves, such as the pulmonary valve, the mitral valve, or the tricuspid valve, and it will be understood that ". Further, the term "proximal," when used in connection with a delivery device or system, refers to a direction relatively close to the user of that device or system when being used as intended, while the term "distal" refers to a direction relatively far from the user of the device. In other words, the leading end of a delivery device or system is positioned distal to a trailing end of the delivery device or system, when being used as intended. As used herein, the terms "substantially," "generally," "approximately," and "about" are intended to mean that slight deviations from absolute are included within the scope of the term so modified. As used herein, the stent may assume an "expanded state" and a "collapsed state," which refer to the relative radial size of the stent.

The present disclosure provides several systems, devices and methods to cut, lacerate, separate or notch a native valve leaflet. The devices and systems described herein may provide a standardized, precise, safe, and fast anterior mitral leaflet laceration procedure that can be performed by unexperienced users (e.g., those inexperienced with LAMPOON and BASILICA procedures). Currently available LAMPOON techniques are complex and long procedures that require thorough preparation of the catheters and the wires used before physicians are ready to lacerate anterior mitral leaflet. In addition, experienced imaging and wire maneuvers are required, and an optimal outcome may not always be possible due to a wide range of anatomic challenges. In a LAMPOON procedure, a native leaflet is first punctured, which may be difficult due to leaflet motion or lack of support. The procedure relies on wires, which may be imprecise and may take multiple attempts before success-a great deal of skill and experience is required to successfully puncture a leaflet. Once the puncture is complete, adjustment of the cutting location is not possible. If the first cut was not long enough (e.g., puncture position was too close to edge of leaflet), then it may be impossible to extend the cut with a second attempt. Additionally, it is often difficult to gauge whether the procedure was successful. Experienced sites need roughly an hour and thirty minutes for a LAMPOON procedure, and this timeframe may be significantly longer in challenging cases. Additionally, as mentioned previously, one of the major disadvantages of a conventional procedure, is that it must be pre-operatively determined which patients have to undergo anterior mitral leaflet laceration using LAMPOON, before a prosthetic mitral valve (e.g., TENDYNE^{™} valve) can be implanted.

Having a device specialized to resolve the above-described challenges (e.g., SAM and SAM-related LVOT obstruction), would make leaflet laceration more reproducible, precise, efficient, and shorter. In addition, the disclosed cutting tool and technique is intended to offer the user the ability to perform and/or revise cutting in advance of a valve replacement, or after valve replacement implantation has occurred to better optimize outcomes with valve implantation. While TENDYNE^{™} TMVI has been noted, the disclosed laceration catheter may be used to improve SAM-related LVOT obstruction outcomes for any mitral valve replacement device including transapical, transseptal, or surgical devices).

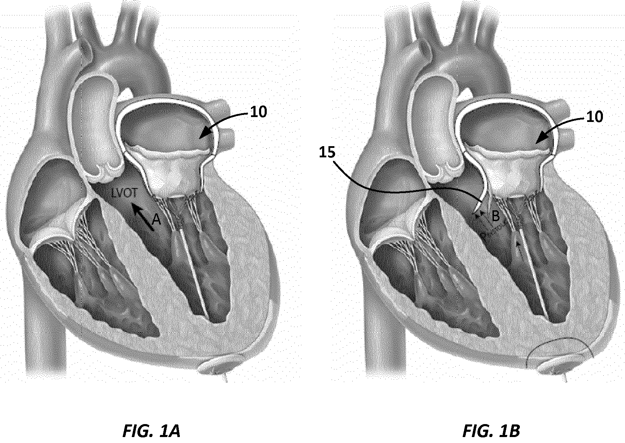

FIG. 1A illustrates normal, unobstructed left ventricular outflow tract (LVOT) flow during cardiac systole. In this example, a prosthetic transcatheter mitral valve 10 has been implanted at the mitral valve annulus. The prosthetic transcatheter mitral valve 10 of FIG. 1A is a tethered valve, but it will be understood that other valve replacement options are also possible. Notice that the installation of a prosthetic mitral valve 10 has little to no effect on blood flow through the left ventricular outflow tract as shown by arrow A. Turning to FIG. 1B, a similar prosthetic transcatheter mitral valve 10 has been implanted, but complications may arise due to the size, shape, length and/or geometry of the anterior mitral leaflet 15. In particular, anterior mitral leaflet 15 may cause left ventricular outflow tract obstruction when systolic pressure pushes the native leaflet (shown by arrow B), causing obstructive Systolic Anterior Motion.

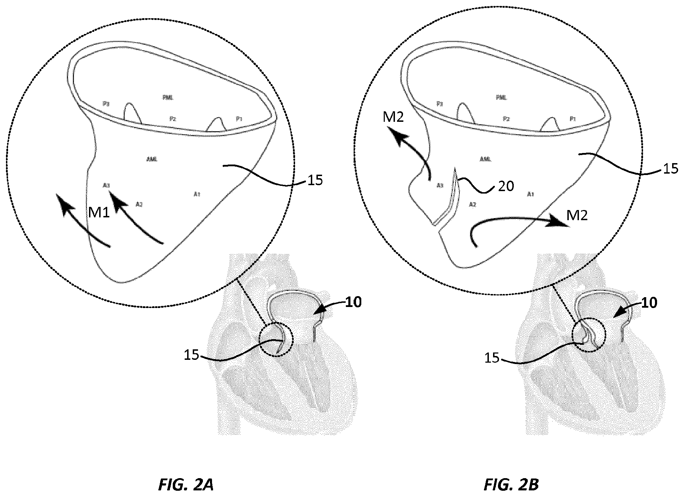

When obstruction is present or expected, as shown in FIG. 1B, one solution is to lacerate the anterior mitral leaflet. This laceration of the mitral leaflet may be performed before, during or after implantation of a prosthetic mitral valve. FIGS. 2A-B illustrate, at a high-level, one example of this laceration. In FIG. 2A, a prosthetic mitral valve 10 has been implanted and an in-tact anterior mitral leaflet 15 actively obstructs, or has the potential to obstruct, the left ventricular outflow tract (LVOT), causing obstructive systolic anterior motion. As shown in FIG. 2A, the anterior mitral leaflet 15 may be conceptually divided into three portions A1, A2, A3. The three portions A1,A2,A3 move together as shown by arrow M1 under systolic pressure.

To resolve, or prevent, this complication, the anterior mitral leaflet 15 may be cut, lacerated or resected as shown in FIG. 2B. Any portion of the anterior mitral leaflet 15 may be cut by the physician as desired. In this example, a single linear or curvilinear laceration 20 is made between portions A2 and A3 of anterior mitral leaflet to separate the distal tip of the anterior mitral leaflet 15 into two parts. Laceration 20 may cause anterior mitral leaflet motion to be less obstructive and reduce or eliminate systolic anterior motion as the leaflets spread laterally. After laceration, portions of the anterior mitral leaflet will separate and move apart as shown by arrow M2.

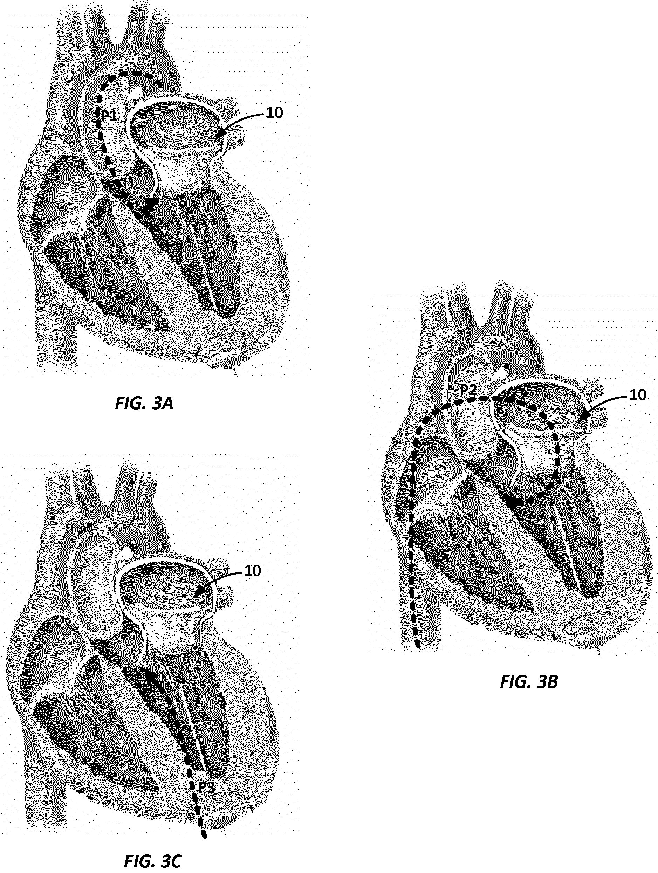

A leaflet laceration device having electrosurgical and/or mechanical laceration capabilities may be used. The leaflet laceration device may allow users to perform laceration of an anterior mitral leaflet using a transapical approach, a transeptal approach before or after valve implantation, or alternatively, retrograde laceration via the aorta before or after valve implantation. FIGS. 3A-C illustrate these certain general approaches. In the cases illustrated, a prosthetic mitral valve has already been implanted in the mitral valve annulus. This may allow operators to evaluate the need for laceration after implantation of a prosthetic valve, or to keep the prosthetic valve in its final position after the implantation while laceration is performed. This tool therefore provides flexibility in a prosthetic mitral valve implantation procedure as retrieval (e.g., bailout due to LVOT obstruction) of such procedures may pose a risk for apical management and/or in damaging or catching on the sub valvular apparatus.

In FIG. 3A, a retrograde approach P1 is shown, in which a transfemoral or transarterial route is taken via the femoral artery, and through the aorta to the anterior mitral leaflet. In FIG. 3B, an antegrade approach P2 is shown, in which a trans-septal route is taken through the femoral vein, then from the right atrium, across the septum to the left atrium, through the mitral valve annulus (or through the implanted prosthetic mitral valve) and around to the anterior mitral leaflet. In FIG. 3C, a transapical approach P3 is shown. The transapical approach may be followed through the same puncture used to implant a prosthetic heart valve, or through a different apical puncture.

Thus, three different feasible approaches (e.g., trans-septal, trans-arterial, and transapical) are disclosed in order to lacerate/cut the anterior mitral leaflet, and each of these approaches has its own advantage. For example, the transapical approach of FIG. 3C may be the most convenient as transapical access is already used for the valve implantation shown. Therefore, adding the step of anterior mitral leaflet laceration may be straightforward. In an alternate case where physicians assess that the best approach is transeptal, the septum would be crossed, and the anterior mitral leaflet grasped and lacerated. In cases where a transeptal procedure is not considered feasible, a transaortic approach would also be possible. This, transaortic approach would also be available as a retrograde bailout approach for post-implantation SAM and LVOT obstruction caused by an anterior mitral leaflet.

In terms of specific benefits, the currently disclosed device and/or approaches may allow or encourage new, less experienced, sites to lacerate anterior mitral leaflets in high-risk SAM patients before a prosthetic valve replacement procedure, and may provide sites a bailout option in case pre-assessment was not entirely accurate and a patient intraoperatively develops SAM. This is particularly beneficial as exact exclusion criteria for patients or AMLs which could possibly develop SAM and LVOT obstruction are not perfectly defined. Thus, having a bailout option is reassuring for many (less experienced) sites and provides flexibility to all prosthetic valve implanters to optimize outcomes without concern for LVOT obstruction.

More specifically, some of the biggest advantages of currently disclosed device and/or approaches include maneuverability, precision, grasping and/or timing. First, maneuverability (e.g., the possibility to grasp an anterior mitral leaflet from different directions and orientations) is of high importance due to anatomical characteristics of each patient. In some instances, it may be desirable to lacerate the anterior mitral leaflet closer to the lateral commissure as sometimes the aorta is positioned laterally. Second, the instant disclosure aims to improve precision and the ability to repeatably lacerate the desired length of the leaflet without cutting too much or too little while avoiding damage to any other anatomical structures (e.g., chords, papillary muscles, and annulus). Because leaflets may vary in length, the depth/length of the laceration/cut may need to be adjusted during the procedure for unique anatomies, and for how an anatomy responds while being lacerated. Third, improved grasping of a native leaflet may provide stability of the leaflet during the laceration/cutting. Finally, the procedure length may be shortened than a traditional procedure.

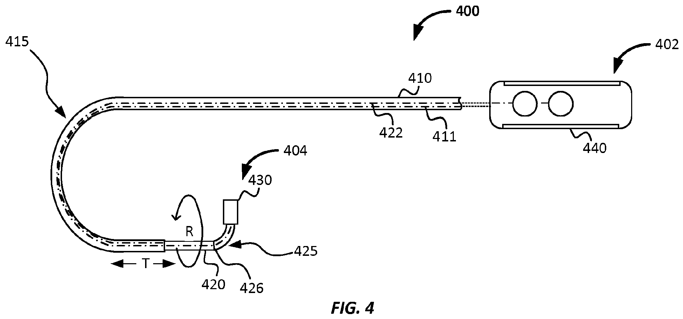

FIG. 4 illustrates a general overview of a medical instrument 400 for performing a leaflet laceration procedure. Generally, instrument 400 may extend between a proximal end 402 and a distal end 404. Instrument 400 may include a flexible outer sheath 410, a steerable inner catheter 420 at least partially disposed within the sheath, and a cutting assembly 430 at the distal tip of the inner catheter for manipulating and/or lacerating a leaflet. As a general overview, cutting assembly 430 may be used to grasp, manipulate or cut the leaflet during the procedure. The inner catheter and the cutting assembly may be steered and actuated via a handle 440 by the operator. In some examples, within the cutting assembly 430 is a cutting tool that facilitates laceration of the leaflet once positioning has been confirmed. This cutting tool may be mechanical (e.g., it may include one or more sharp blades) or electrical (e.g., it may include wire in electrical communication with an electrosurgical generator) in nature. The physician may perform the leaflet laceration procedure with instrument 400 by navigating the sheath toward the target leaflet, grasping a leaflet using x-ray and/or echo imaging and activating the cutting mechanism. Depending on the desired approach, the sheath 410 and/or the inner catheter 420 may be flexible or deflectable to bend and pass through the aortic arch as shown with arrow 415, and the inner catheter 420 may also be deflectable in the annular plane as shown with arrow 425. To aid in the deflection in the annular plane, inner catheter 420 may include a deflecting member or hinge point 424 proximal to cutting assembly 430 to achieve a bend of approximately 90 degrees as shown. Inner catheter 420 may also be translatable as shown with arrow "T" and/or rotatable as shown with arrow "R" with respect to sheath 410.

In some examples, the outer sheath 410 is long enough to cross the aortic arch via transfemoral access. This may allow the physician to easily track the device to the annulus and allow navigation anteriorly and/or posteriorly within the annulus. One or more pull wires 411 may couple to the distal end of the sheath 410 and extend toward the handle 440 to allow the physician to control the sheath (e.g., to actively flex or bend it through the aortic arch). Within outer sheath 410, the inner catheter 420 may also easily track the anatomy and translate and/or rotate independently within the sheath 410. The deflectable section or hinge point 426 at arrow 425 may allow the cutting assembly to deflect parallel to the annular plane for leaflet grasping. In some examples, inner catheter 420 has one or more pull wires 422 running through it from its distal end to handle 440 that allow the physician to actuate the cutting assembly from the handle 440.

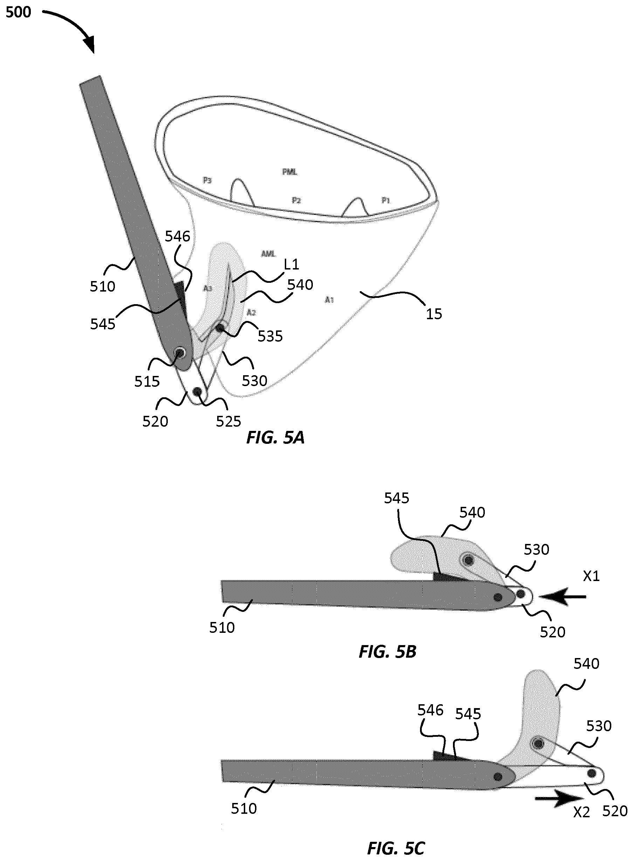

FIGS. 5A-C illustrate a first example of a leaflet laceration device 500. A retrograde transfemoral approach is shown in this example, and the laceration device 500 is being shown as cutting the ventricular surface of an anterior mitral leaflet 15. As shown, laceration device 500 includes an elongated shaft 510 coupled to a central actuator comprised of two linkages. A first intermediate linkage 520 is coupled to shaft 510 via a first pin 515, the first intermediate linkage 520 being coupled to a second intermediate linkage 530 via a second pin 525. The second intermediate linkage 530 may be coupled to a moveable arm 540 via a third pin 535 capable of grasping, pushing and/or manipulating the leaflet. The central actuator (e.g., first and second intermediate linkages 520,530) may be pivotably connected to elongated shaft 510. A blade 545 having a curved or linear sharp edge 546 may be disposed adjacent, and coupled to elongated shaft 510 opposite the arm 540. The central actuator, and specifically first intermediate linkage 520 may be translatable relative to elongated shaft 510 so that the first intermediate linkage 520 may be pulled proximally in direction of arrow X1 to place the device in a closed condition and cut tissue against blade 545 (FIG. 5B). Conversely, the first intermediate linkage 520 may be advanced distally in the direction of arrow X2 to transition the device to an open condition and release and/or re-grasp tissue (FIG. 5C). In FIG. 5A, a longitudinal slit or laceration L1 that would result from use of the device is shown. In some examples, first pin 515 may be in a welded half-pin configuration to allow for pivoting and/or translation of the first intermediate linkage 520 with respect to elongated shaft 510. As shown, the elongated shaft 510 will be advanced adjacent the anterior mitral leaflet, and blade 545, disposed near the anterior mitral leaflet, will first contact and cut through on the ventricular surface of the leaflet as the arm 540 presses the leaflet against blade 545.

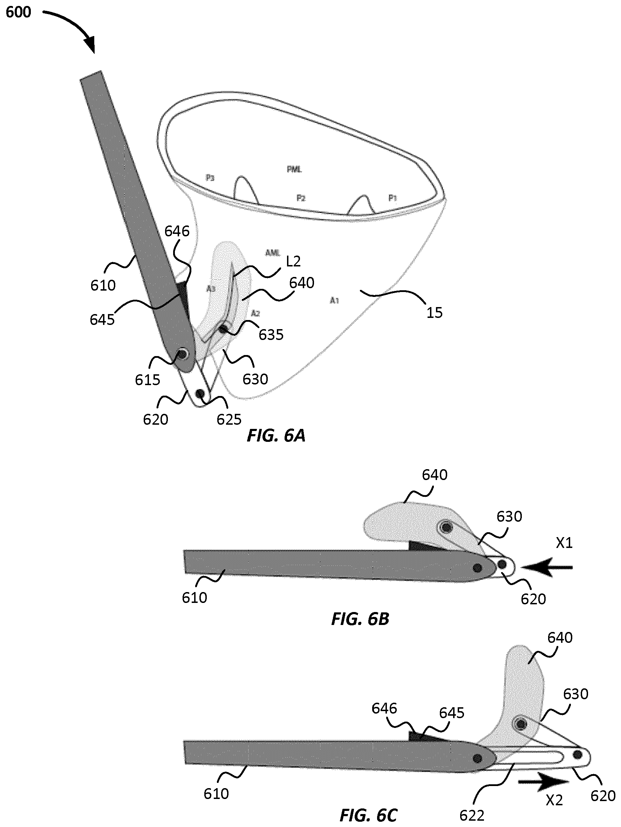

FIGS. 6A-C illustrate a second example of a leaflet laceration device 600, similar to leaflet laceration device 500. A retrograde transfemoral approach is shown in this example, and the laceration device 600 is being shown as cutting the ventricular surface of an anterior mitral leaflet 15. As shown, laceration device 600 includes an elongated shaft 610 coupled to a central actuator comprised of a first intermediate linkage 620 via a first pin 615, the first intermediate linkage 620 being coupled to a second intermediate linkage 630 via a second pin 625. The second intermediate linkage 630 may be coupled to an arm 640 via a third pin 635. A blade 645 having a curved or linear sharp edge 646 may be disposed and coupled to elongated shaft 610 opposite the arm 640. In this example, first pin 615 is configured as a solid thru-pin, and first intermediate linkage 610 includes an elongated slot 622 through which first pin 615 may travel. Leaflet laceration device 600 may transition between open and closed conditions to cut the ventricular surface of an anterior mitral leaflet with blade 645 and produce a laceration L2 (FIG. 6A).

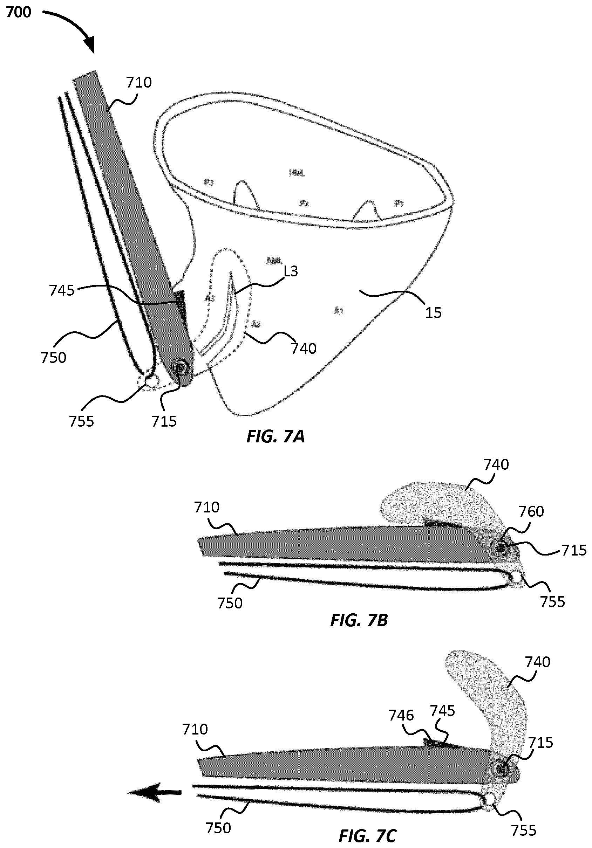

In a third example, shown in FIGS. 7A-C, a spring-loaded leaflet laceration device 700 is shown. A retrograde transfemoral approach is shown in this example, and the laceration device 700 is being shown as cutting the ventricular surface of an anterior mitral leaflet 15. Similar to the previous embodiments, laceration device 700 includes an elongated shaft 710, but the shaft is directly coupled to moveable arm 740 via a first pin 715. The actuating mechanism of laceration device 700 includes a pull-hole 755 disposed on the proximal end of the arm 740, and a pull-wire 750 extending along the length of the elongated shaft 710 and looping through pull-hole 755. A blade 745 having a curved or linear sharp edge 746 may be disposed and coupled to elongated shaft 710 opposite the arm 740. A torsional spring 760 may be disposed adjacent or integrated within first pin 715 and may be configured to bias the arm 740 into the closed condition (FIG. 7B) absent an external force. Conversely, when a tension is applied to pull-wire 750, it may actuate proximal end of arm 740 to overcome the torsional spring force and transition the device into the open condition (FIG. 7C). Once the pull-wire is released, the torsional spring 760 will urge the arm back toward blade 745 and lacerate the leaflet against sharp edge 746 of the blade. In this example, as with others, a mechanical sharp edge is used to lacerate the leaflet. It will be understood, however, that a traditional radiofrequency electrode or radiofrequency cautery tool (e.g., electrosurgical wire) may be used to lacerate the tissue instead of a mechanical blade.

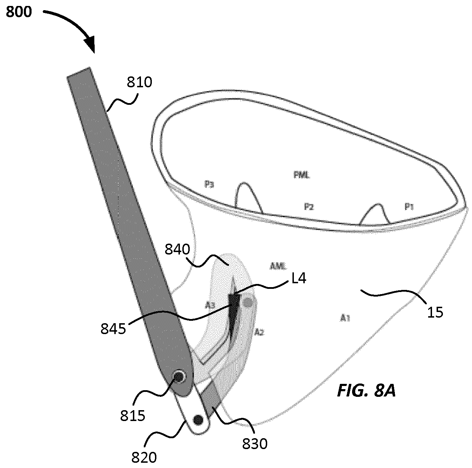

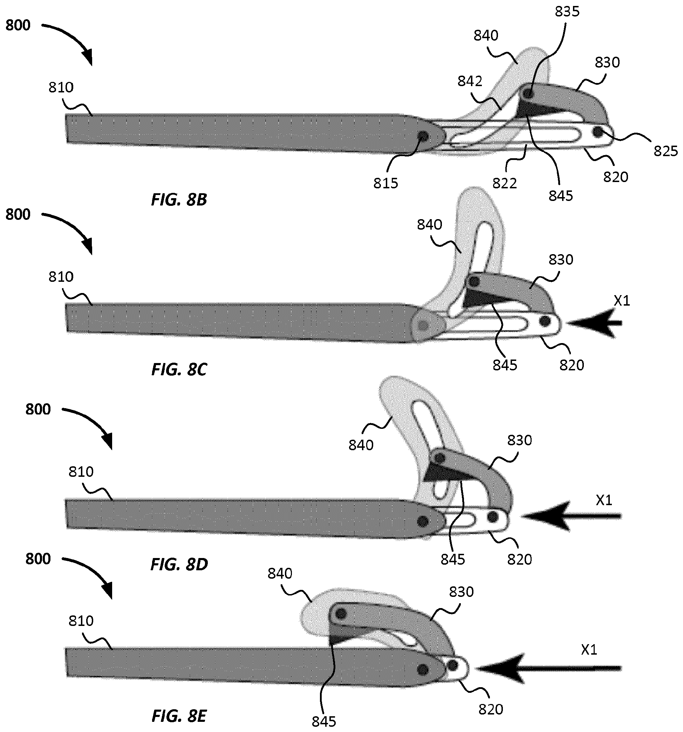

In a fourth example, shown in FIGS. 8A-E, a leaflet laceration device 800 is shown. A retrograde transfemoral approach is again illustrated, but the laceration device 800 is being shown as cutting the atrial surface of an anterior mitral leaflet 15. As shown, laceration device 800 includes an elongated shaft 810 coupled to a central actuator comprised of a first intermediate linkage 820 via a first pin 815, the first intermediate linkage 820 being coupled to a second curved linkage 830 via a second pin 825. The second curved linkage 830 may be coupled to a moveable arm 840 via a third pin 835. A blade 845 having a sharp edge is coupled to second curved linkage 830 with the sharp edge pointing back toward elongated shaft 810. In this example, first intermediate linkage 820 includes a longitudinal slot 822 that serves as a guide for pin 815, and arm 840 includes a curved slot 842 that serves as a guide for pin 835. The first intermediate linkage 820 may translate relative to elongated shaft 810, while arm 840 may rotate with respect to the elongated shaft 810 when transitioning between the fully open condition (FIG. 8B) and the fully closed condition (FIG. 8E). Specifically, the first intermediate linkage 820 may be pulled toward the elongated shaft 810 in the direction of arrow X1 to urge the device 800 to the closed condition, and may be pushed in a direction opposite arrow X1 to urge the device 800 to the open condition. In this example, blade 845 may be only exposed to the native leaflet in the fully closed condition of FIG. 8E.

In use, the surgeon may perform a leaflet laceration procedure using either the mechanical or an electrosurgical variation. In either case, the medical instrument may be advanced to the target site via one of the approaches suggest in FIGS. 3A-C. For example, the laceration device may be steered through the femoral artery up toward, and around, the aortic arch and down through the aortic annulus and to the anterior mitral leaflet. Once in position, the moveable arm may manipulate the leaflet and cutting assembly may perform the laceration on a desired surface. In the mechanical variation, the arm may press the leaflet against a blade disposed on opposite side of the leaflet to lacerate it (*See,* FIGS. 5A-7C). Alternatively, the arm and the blade may be disposed on a same side of the leaflet and the arm may urge the blade across the leaflet to lacerate it (*See,* FIGS. 8A-E). As noted, an electrosurgical variation is possible. In this example, the procedure may be identical to that described, until the cutting step, where the electrosurgical wire would be used to contact the leaflet to lacerate it.

In some examples, a method of treatment includes providing a leaflet laceration device including an elongated shaft, a moveable arm coupled to the elongated shaft and pivotable relative thereto between an open condition and a closed condition, and a cutting element coupled to at least one of the elongated shaft and the moveable arm, advancing the leaflet laceration device to an anterior mitral leaflet, and cutting the anterior mitral leaflet with the cutting element. The method may further include advancing the leaflet laceration device to an anterior mitral leaflet comprises steering the leaflet laceration device through a femoral artery, via and through an aorta, to the anterior mitral leaflet, and/or advancing the leaflet laceration device to an anterior mitral leaflet, which includes steering the leaflet laceration device through a femoral vein, a right atrium, across a septum to a left atrium and through a previously implanted prosthetic valve to the anterior mitral leaflet, and/or advancing the leaflet laceration device to an anterior mitral leaflet which includes advancing the leaflet laceration device through an apex of a heart, and/or the step of implanting a prosthetic heart valve prior to advancing the leaflet laceration device to the anterior mitral leaflet, and/or the step of implanting a prosthetic heart valve after advancing the leaflet laceration device to the anterior mitral leaflet.

It is to be understood that the embodiments described herein are merely illustrative of the principles and applications of the present disclosure. For example, a system may be battery-operated, or the handle and generator may be integrated. Additionally, a system may include both mechanical and electrical cutting elements. Moreover, certain components are optional, and the disclosure contemplates various configurations and combinations of the elements disclosed herein. It is therefore to be understood that numerous modifications may be made to the illustrative embodiments and that other arrangements may be devised without departing from the scope of the present disclosure. The invention is defined by the appended claims.

## Claims

1. A leaflet laceration device, comprising:
an elongated shaft (710); and
a moveable arm (740) coupled to the elongated shaft (710) and pivotable relative thereto between an open condition and a closed condition;
**characterized in that** a cutting element is coupled to the elongated shaft (710) opposite the moveable arm (740), the cutting element being configured and arranged to contact a first surface of an anterior mitral leaflet (15).

2. The leaflet laceration device of claim 1, wherein the cutting element is configured to lacerate a ventricular surface of the anterior mitral leaflet (15).

3. The leaflet laceration device of claim 2, further comprising a first intermediate linkage (520) coupled to the elongated shaft (710) via a first pin (615), and a second intermediate linkage (530) coupled to the first intermediate linkage (520) via a second pin (625) and to the moveable arm (740) via a third pin (635).

4. The leaflet laceration device of claim 3, wherein the first intermediate linkage (520) is translatable relative to the elongated shaft (710).

5. The leaflet laceration device of claim 4, wherein the first intermediate linkage (520) defines an elongated slot (622), the first pin (615) being movable within the elongated slot (622).

6. The leaflet laceration device of claim 2, wherein the movable arm (740) defines a pull-hole (755), and further comprising a pull-wire (750) coupled through the pull-hole (755) and extending parallel with the elongated shaft (710).

7. The leaflet laceration device of claim 6, wherein actuating the pull-wire (750) transitions the moveable arm (740) to the open condition.

8. The leaflet laceration device of claim 6, further comprising a spring (760) configured to keep the moveable arm (740) in the closed condition when no tension is applied to the pull-wire (750).

9. The leaflet laceration device of claim 1, wherein the cutting element comprises a blade (745) having a sharp edge (746).

10. The leaflet laceration device of claim 1, wherein the cutting element comprises an electrosurgical wire in electrical communication with a generator.

## Patentansprüche

1. Klappensegel-Lazerationsvorrichtung, umfassend:
einen langgestreckten Schaft (710); und
einen bewegbaren Arm (740), der mit dem langgestreckten Schaft (710) gekoppelt und zwischen einem offenen Zustand und einem geschlossenen Zustand relativ dazu schwenkbar ist;
**dadurch gekennzeichnet, dass** ein Schneidelement mit dem langgestreckten Schaft (710) gegenüber dem bewegbaren Arm (740) gekoppelt ist, wobei das Schneidelement dazu ausgebildet und angeordnet ist, eine erste Oberfläche eines anterioren Mitralsegels (15) zu kontaktieren.

2. Klappensegel-Lazerationsvorrichtung nach Anspruch 1, wobei das Schneidelement dazu ausgebildet ist, eine ventrikuläre Oberfläche des anterioren Mitralsegels (15) zu lazerieren.

3. Klappensegel-Lazerationsvorrichtung nach Anspruch 2, weiter umfassend ein erstes Zwischenglied (520), das über einen ersten Stift (615) mit dem langgestreckten Schaft (710) gekoppelt ist, und ein zweites Zwischenglied (530), das über einen zweiten Stift (625) mit dem ersten Zwischenglied (520) und über einen dritten Stift (635) mit dem bewegbaren Arm (740) gekoppelt ist.

4. Klappensegel-Lazerationsvorrichtung nach Anspruch 3, wobei das erste Zwischenglied (520) relativ zu dem langgestreckten Schaft (710) verschiebbar ist.

5. Klappensegel-Lazerationsvorrichtung nach Anspruch 4, wobei das erste Zwischenglied (520) ein Langloch (622) definiert, wobei der erste Stift (615) innerhalb des Langlochs (622) bewegbar ist.

6. Klappensegel-Lazerationsvorrichtung nach Anspruch 2, wobei der bewegbare Arm (740) ein Zugloch (755) definiert, und weiter umfassend einen Zugdraht (750), der durch das Zugloch (755) hindurch gekoppelt ist und sich parallel zu dem langgestreckten Schaft (710) erstreckt.

7. Klappensegel-Lazerationsvorrichtung nach Anspruch 6, wobei das Betätigen des Zugdrahts (750) den bewegbaren Arm (740) in den offenen Zustand überführt.

8. Klappensegel-Lazerationsvorrichtung nach Anspruch 6, weiter umfassend eine Feder (760), die dazu ausgebildet ist, den bewegbaren Arm (740) im geschlossenen Zustand zu halten, wenn keine Spannung an den Zugdraht (750) angelegt wird.

9. Klappensegel-Lazerationsvorrichtung nach Anspruch 1, wobei das Schneidelement eine Klinge (745) mit einer scharfen Schneide (746) umfasst.

10. Klappensegel-Lazerationsvorrichtung nach Anspruch 1, wobei das Schneidelement einen elektrochirurgischen Draht in elektrischer Verbindung mit einem Generator umfasst.

## Revendications

1. Dispositif de lacération de feuillet, comprenant :
une tige allongée (710) ; et
un bras mobile (740) couplé à la tige allongée (710) et pivotant par rapport à celle-ci entre une condition ouverte et une condition fermée ;
**caractérisé en ce qu'**un élément de coupe est couplé à la tige allongée (710) à l'opposé du bras mobile (740), l'élément de coupe étant configuré et agencé pour venir en contact avec une première surface d'un feuillet mitral antérieur (15).

2. Dispositif de lacération de feuillet selon la revendication 1, dans lequel l'élément de coupe est configuré pour lacérer une surface ventriculaire du feuillet mitral antérieur (15).

3. Dispositif de lacération de feuillet selon la revendication 2, comprenant en outre une première liaison intermédiaire (520), couplée à la tige allongée (710) par l'intermédiaire d'une première goupille (615), et une seconde liaison intermédiaire (530) couplée à la première liaison intermédiaire (520) par l'intermédiaire d'une deuxième goupille (625) et au bras mobile (740) par l'intermédiaire d'une troisième goupille (635).

4. Dispositif de lacération de feuillet selon la revendication 3, dans lequel la première liaison intermédiaire (520) est mobile en translation par rapport à la tige allongée (710).

5. Dispositif de lacération de feuillet selon la revendication 4, dans lequel la première liaison intermédiaire (520) définit une lumière allongée (622), la première goupille (615) étant mobile à l'intérieur de la lumière allongée (622).

6. Dispositif de lacération de feuillet selon la revendication 2, dans lequel le bras mobile (740) définit un orifice de traction (755), et comprenant en outre un fil de traction (750) couplé à travers l'orifice de traction (755) et s'étendant parallèlement à la tige allongée (710).

7. Dispositif de lacération de feuillet selon la revendication 6, dans lequel l'actionnement du fil de traction (750) fait passer le bras mobile (740) dans la condition ouverte.

8. Dispositif de lacération de feuillet selon la revendication 6, comprenant en outre un ressort (760) configuré pour maintenir le bras mobile (740) dans la condition fermée lorsqu'aucune tension n'est appliquée au fil de traction (750).

9. Dispositif de lacération de feuillet selon la revendication 1, dans lequel l'élément de coupe comprend une lame (745) présentant un bord tranchant (746).

10. Dispositif de lacération de feuillet selon la revendication 1, dans lequel l'élément de coupe comprend un fil électrochirurgical en communication électrique avec un générateur.
